# EUROPEAN PATENT APPLICATION

(11) **EP 3 900 691 A1**
(43) Date of publication of application: **27.10.2021**
(21) Application number: 20900727.7
(22) Date of filing: 29.10.2020
(51) Int. Cl.: A61H 3/00, A61F 5/01, A61F 5/10

(54) **SOLE SUPPORT**

(30) Priority: 03.02.2020 JP 2020027567
(71) Applicant: Robanomimi Inc., Sendai-shi, Miyagi 980-0801 (JP)
(72) Inventor: SUGAWARA, Ryo, Sendai-shi, Miyagi 980-0801 (JP)
(74) Representative: Winter, Brandl - Partnerschaft mbB
(86) International application number: PCT/JP2020/040563
(87) International publication number: WO 2021/157143

(57) **Abstract**

A sole support (10) constitutes at least a part of a support surface that supports a sole. The sole support (10) includes a high portion (12) that constitutes a part of the support surface under at least a part of a distal phalange (BN1) at a higher position than a part of the support surface under a proximal joint of a proximal phalange (BN3) for each toe so that a proximal joint of the distal phalange (BN1) is above the proximal joint of the proximal phalange (BN3) in each toe.

## Description

### Technical Field

The present invention relates to a sole support.

### Background Art

As described in Patent Document 1, a three-point support type toe orthosis used for a toe with a fracture in a distal phalange is known. The toe orthosis includes a proximal portion that contacts the toe under a middle phalange, a distal portion that contacts the toe under the distal phalange, and a toe backrest that contacts the toe over a proximal joint (in other words, a joint between the middle phalange and the distal phalange) of the distal phalange. This allows a state in which the proximal joint of the distal phalange of the toe with the fracture in the distal phalange is extended to be maintained.

### Citation List

### Patent Literature

Patent Literature 1: Japanese Registered Utility Model No. 3103366

### Summary of Invention

### Technical Problem

Toe deformities, such as hammer toe and claw toe, are well known. In the toe deformity, at least one of the distal joints of the proximal phalanges of the first toe to the fifth toe may bend excessively. The distal joint of the proximal phalange of the first toe is the joint between the proximal phalange and the distal phalange in the first toe. The distal joint of the proximal phalange of one of the second toe to the fifth toe is the joint between the proximal phalange and the middle phalange.

Therefore, it is conceivable to use the above toe orthosis for a toe with the toe deformity. However, when the above toe orthosis is used, the distal joint of the proximal phalange is fixed. This also tends to reduce the degree of freedom of motions of joints in the vicinity of the above joint. In addition, during exercise (e.g., walking, pedaling, etc.), the force applied to the tips of the toes with the toe deformity tends to cause pain. As a result, when a user with the toe deformity uses the above toe orthosis, the user has a difficulty to exercise.

One of the purposes of this invention is to make it easier for a user with a toe deformity to exercise when using.

### Solution to Problem

In one aspect, a sole support constitutes at least a part of a support surface that supports a sole. The sole support includes a high portion that constitutes a part of the support surface under at least a part of a distal phalange at a higher position than a part of the support surface under a proximal joint of a proximal phalange for each toe so that a proximal joint of the distal phalange is above the proximal joint of the proximal phalange in each toe.

### Advantageous Effects of Invention

The use of the sole support by the user with the toe deformity makes it easier for the user to exercise.

### Brief Description of Drawings

Fig. 1 is a perspective view of a sole support and a sole part of a first embodiment.
Fig. 2 is a plan view of the sole support and the sole part of the first embodiment.
Fig. 3 is a partial cross-sectional view of the sole support and the sole part of the first embodiment.
Fig. 4 is a perspective view of the sole support of the first embodiment.
Fig. 5 is a perspective view of the sole support of the first embodiment.
Fig. 6 is a plan view of the sole support of the first embodiment.
Fig. 7 is a bottom view of the sole support of the first embodiment.
Fig. 8 is a front view of the sole support of the first embodiment.
Fig. 9 is a back view of the sole support of the first embodiment.
Fig. 10 is a right side view of the sole support of the first embodiment.
Fig. 11 is a left side view of the sole support of the first embodiment.
Fig. 12 is a perspective view of a sole support of a second embodiment.
Fig. 13 is a plan view of the sole support of the second embodiment.
Fig. 14 is a bottom view of the sole support of the second embodiment.
Fig. 15 is a front view of the sole support of the second embodiment.
Fig. 16 is a back view of the sole support of the second embodiment.
Fig. 17 is a right side view of the sole support of the second embodiment.
Fig. 18 is a left side view of the sole support of the second embodiment.
Fig. 19 is a side view of a three-dimensional mallet toe corrector of a third embodiment.
Fig. 20 is a top view of the three-dimensional mallet toe corrector for a left foot of the third embodiment.

### Description of Embodiments

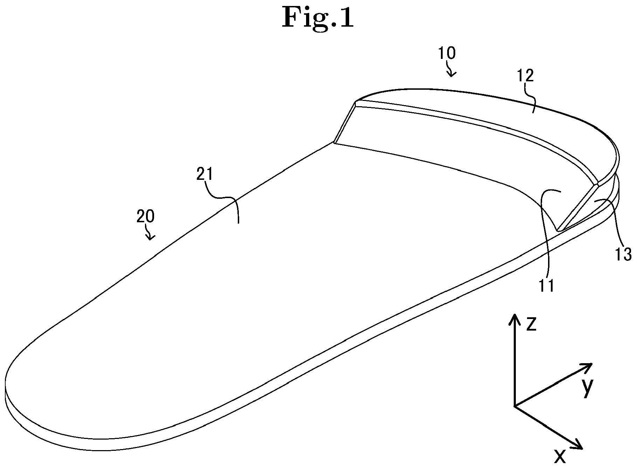

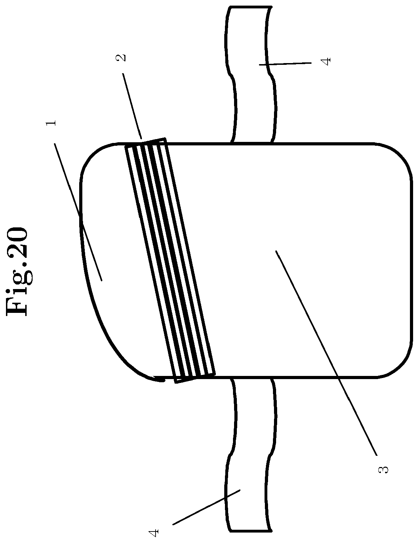

Hereinafter, each embodiment of the sole support of the present invention will be described with reference to Figs. 1 to 20.

### <First Embodiment>

### (Overview)

A sole support of a first embodiment constitutes at least a part of a support surface that supports a sole.

The sole support includes a high portion that constitutes a part of the support surface under at least a part of a distal phalange at a higher position than a part of the support surface under a proximal joint of a proximal phalange for each toe so that a proximal joint of the distal phalange is above the proximal joint of the proximal phalange in each toe.

According to this, the proximal joint of the distal phalange is located above the proximal joint of the proximal phalange for each toe. This makes it possible to bring the distal joint of the proximal phalange for each toe closer to a state in which the distal joint is extended. Further, a ratio of a force applied to the tip portion of each toe to a total force applied to the sole can be reduced.

Thus, according to the sole support, an excessive force to be applied to the tip portion of each toe in a state where at least one of the distal joints of the proximal phalange of each toe is excessively bent can be avoided. Further, as compared with the case where the distal joint of the proximal phalange of each toe is fixed, the degree of freedom of motions of the distal joint and another joint in the vicinity of the distal joint can be increased. As a result, the user with the toe deformity can easily exercise by using the sole support.

Next, the sole support of the first embodiment will be described in detail with reference to Figs. 1 to 11.

### (Configuration)

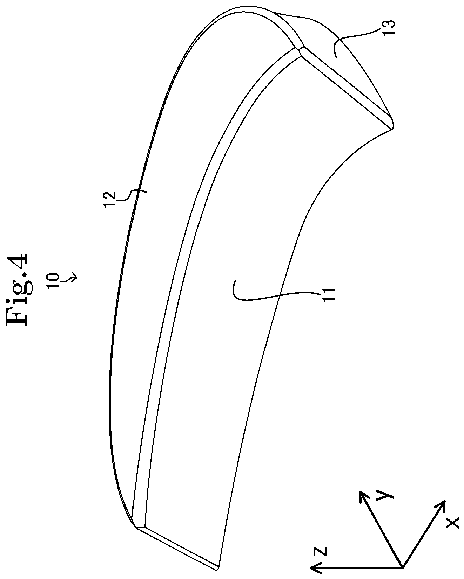

Hereinafter, the sole support 10 of the first embodiment will be described using a right-handed orthogonal coordinate system having an x-axis, a y-axis, and a z-axis, as illustrated in Figs. 1 to 3. In this specification, the same coordinate system is used in Figs. 4 to 18 described later.

In the present specification, the sole support 10 for a left foot will be described. Since the sole support 10 for a right foot is symmetrical with the sole support 10 for the left foot, the description thereof is omitted.

In this example, the x-axis direction, the y-axis direction, and the z-axis direction may be represented as the left-right direction of the sole support 10, the front-back direction of the sole support 10, and the up-down direction of the sole support 10, respectively. In this example, the x-axis positive direction, the x-axis negative direction, the y-axis positive direction, the y-axis negative direction, the z-axis positive direction, and the z-axis negative direction are the right direction of the sole support 10, the left direction of the sole support 10, the front direction of the sole support 10, the back direction of the sole support 10, the up direction of the sole support 10, and the down direction of the sole support 10, respectively.

In this example, the z-axis positive direction and the z-axis negative direction coincide with the vertically upward direction and the vertically downward direction, respectively.

As illustrated in Figs. 1 to 3, the sole support 10 of the first embodiment is fixed to a top surface 21 which is an end surface of a sole part 20 in the vertically upward direction. In this specification, the vertically upward direction and the vertically downward direction are also represented as the up direction and the down direction, respectively.

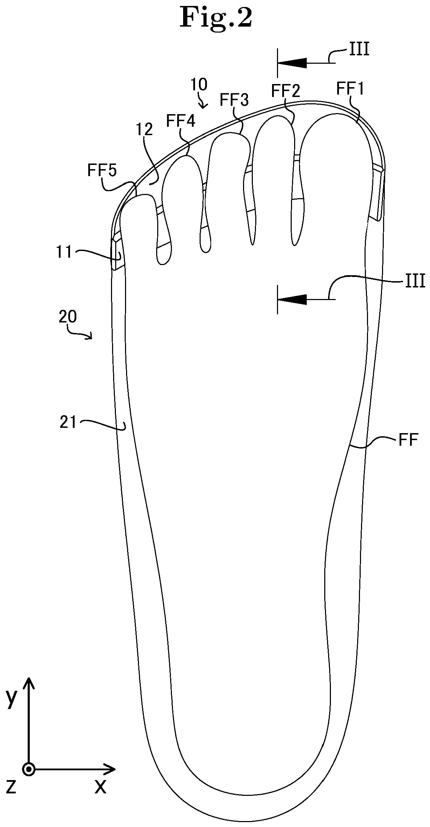

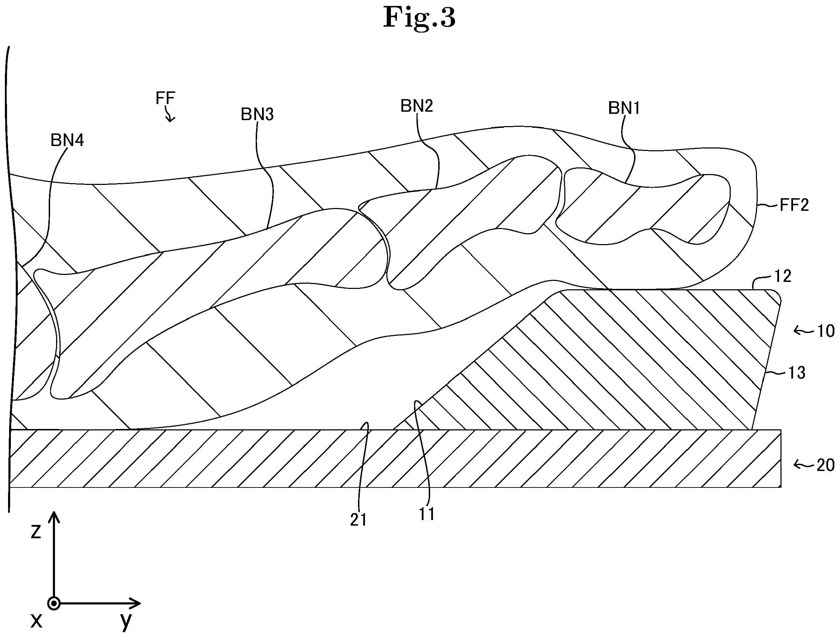

Fig. 1 is a view (in other words, a right back upper perspective view) of the sole support 10 and the sole part 20 from a position in right of the sole support 10, in back of the sole support 10, and above the sole support 10. Fig. 2 is a view (in other words, a plan view) of the sole support 10 and the sole part 20 from a position above the sole support 10. Fig. 3 is a partial cross-sectional view of the sole support 10 and the sole part 20 cut by a plane represented by a III-III line of Fig. 2 seen in the x-axis negative direction. Figs. 2 and 3 illustrate a state (in other words, a foot resting state) in which the foot FF is placed on the sole support 10 and the sole part 20.

In this example, the top surface 21 together with the sole support 10 constitutes a support surface that supports the sole of the user. The sole support 10 may be represented as a toe support or a toe corrector.

In this example, the sole part 20 constitutes the bottom layer of an orthosis, such as a shoe type orthosis, or a leg orthosis. For example, the sole support 10 may be used as a motion auxiliary to assist the exercise by the user with the toe deformity. For example, the sole support 10 may be used as a corrector for correcting the toe of the toe deformity.

The sole part 20 may constitute the bottom layer of a footwear such as a shoe, a slipper, or a sandal. Alternatively, the sole part 20 may constitute an insole (in other words, an insole or a foot bed) of the orthosis or the footwear. Also, the sole part 20 may constitute a pedal in an exercise equipment used in a rehabilitation or in an exercise equipment simulating an exercise such as walking or pedaling a bicycle.

In this example, the top surface 21 is a plane that coincides with the horizontal plane. Note that the top surface 21 may have unevenness along unevenness of at least the part of the sole.

In this example, the sole support 10 is fixed to the top surface 21 with an adhesive. The sole support 10 may be fixed to the top surface 21 with a fastener such as a screw in addition to the adhesive or instead of the adhesive. Alternatively, the sole support 10 may be fixed to the top surface 21 with a band-shaped or string-shaped coupler in addition to the adhesive or instead of the adhesive.

The sole support 10 may be integrally formed with the sole part 20.

As illustrated in Fig. 2, the sole support 10 is located under the tip portion of the foot FF in the top surface 21 in the foot resting state. In this example, in the foot resting state, the sole support 10 is located in the top surface 21 under the first toe FF1, the second toe FF2, the third toe FF3, the fourth toe FF4, and the fifth toe FF5 of the foot FF.

As illustrated in Figs. 4 to 11, the sole support 10 is three-dimensional. In this example, the sole support 10 is solid.

In this example, the sole support 10 is made of a material having rubber elasticity. For example, the sole support 10 is made of natural rubber, synthetic rubber, synthetic resin, or elastomer. In this example, the sole support 10 is made of silicone rubber. Alternatively, the sole support 10 may be made of a material having no rubber elasticity (for example, synthetic resin or metal). The sole support 10 may be made of a plurality of materials. In this case, the sole support 10 may have a plurality of layers composed of a plurality of materials, respectively. Also, at least a part (for example, a part in contact with the sole) of the surface of the sole support 10 may be covered with a covering material such as fiber.

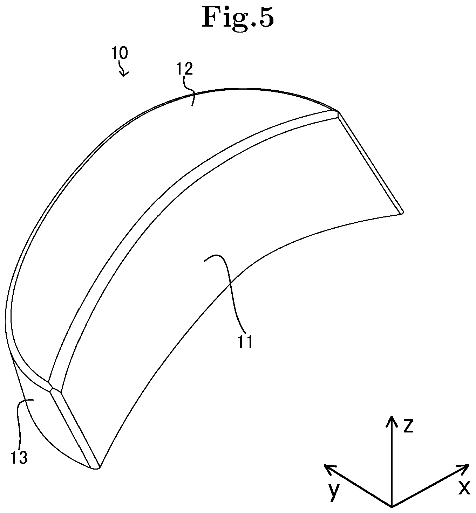

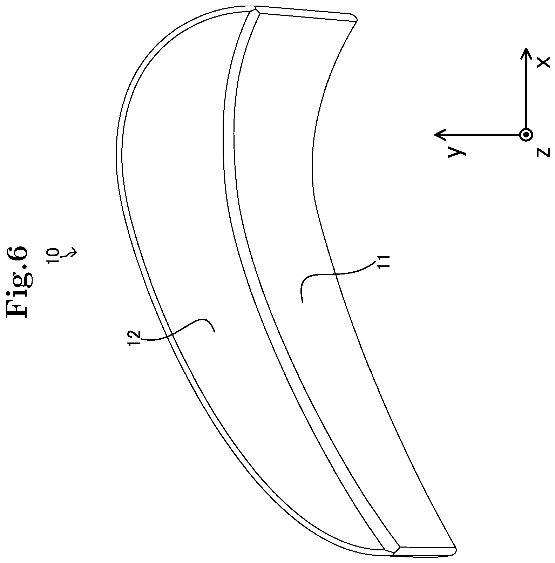

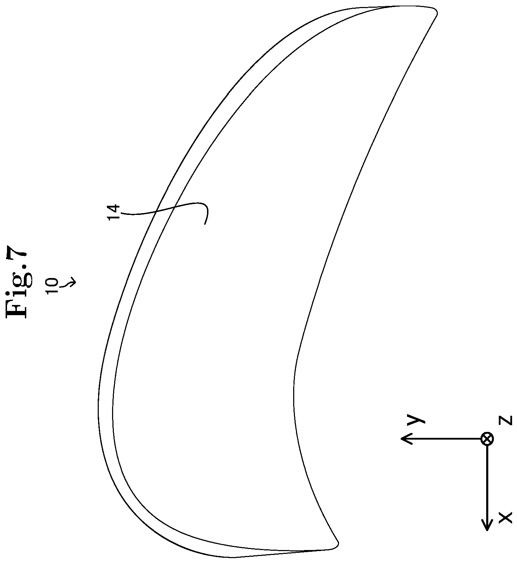

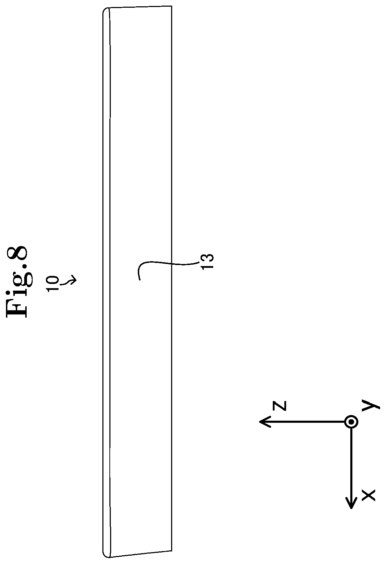

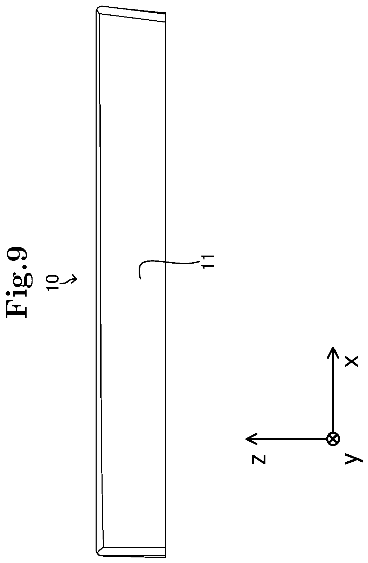

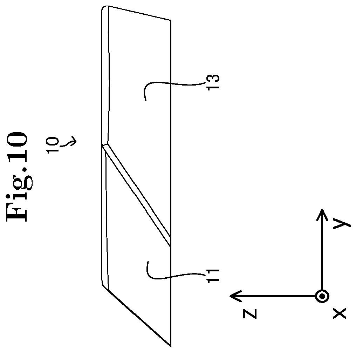

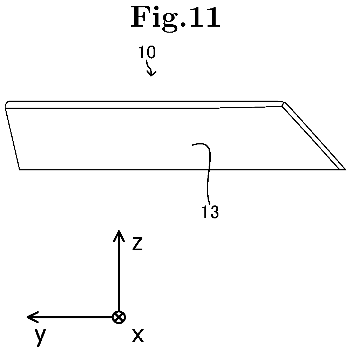

Fig. 4 is a right back upper perspective view of the sole support 10. Fig. 5 is a view (in other words, a left back upper perspective view) of the sole support 10 from a position in left of the sole support 10, in back of the sole support 10, and above the sole support. Fig. 6 is a plan view of the sole support 10. Fig. 7 is a view (in other words, bottom view) of the sole support 10 from a position below the sole support 10. Fig. 8 is a view (in other words, front view) of the sole support 10 from a position in front of the sole support 10. Fig. 9 is a view (in other words, back view) of the sole support 10 from a position in back of the sole support 10. Fig. 10 is a view (in other words, right side view) of the sole support 10 from a position in right of the sole support 10. Fig. 11 is a view (in other words, left side view) of the sole support 10 from a position in left of the sole support 10.

As illustrated in Figs. 4 to 11, the sole support 10 includes a back surface 11 which constitutes the end surface in the y-axis negative direction, a top surface 12 which constitutes the end surface in the z-axis positive direction, a front surface 13 that constitutes the end surface in the y-axis positive direction, and a bottom surface 14 that constitutes the end surface in the z-axis negative direction.

As illustrated in Fig. 6, the top surface 12 has a crescent shape extending along the x-axis direction. Each of the end (in other words, the front edge of the top surface 12) of the top surface 12 in the y-axis positive direction and the end (in other words, the back edge of the top surface 12) of the top surface 12 in the y-axis negative direction has a shape that curves so that the center in the x-axis direction protrudes toward the y-axis positive direction more than the both ends in the x-axis direction.

As illustrated in Fig. 7, the bottom surface 14 has a crescent shape extending along the x-axis direction. Each of the end (in other words, the front edge of the bottom surface 14) of the bottom surface 14 in the y-axis positive direction and the end (in other words, the back edge of the bottom surface 14) of the bottom surface 14 in the y-axis negative direction has a shape that curves so that the center in the x-axis direction protrudes toward the y-axis positive direction more than the both ends in the x-axis direction.

As illustrated in Figs. 6 and 7, the length of the top surface 12 in the y-axis direction is shorter than the length of the bottom surface 14 in the y-axis direction. The back edge of the top surface 12 has a more positive position in the y-axis direction than the back edge of the bottom surface 14. In this example, the front edge of the top surface 12 has a more positive position in the y-axis direction than the front edge of the bottom surface 14.

As illustrated in Figs. 4 to 6, the back surface 11 connects the back edge of the top surface 12 and the back edge of the bottom surface 14. In other words, the end (in other words, the upper edge of the back surface 11) of the back surface 11 in the z-axis positive direction coincides with the back edge of the top surface 12, and the end (in other words, the lower edge of the back surface 11) of the back surface 11 in the z-axis negative direction coincides with the back edge of the bottom surface 14.

As illustrated in Figs. 4, 8 and 10, the front surface 13 connects the front edge of the top surface 12 and the front edge of the bottom surface 14. In other words, the end (in other words, the upper edge of the front surface 13) of the front surface 13 in the z-axis positive direction coincides with the front edge of the top surface 12, and the end (in other words, the lower edge of the front surface 13) of the front surface 13 in the z-axis negative direction coincides with the front edge of the bottom surface 14.

In this example, as illustrated in Figs. 4 to 6, the boundary (in other words, the connection portion) between the back surface 11 and the top surface 12, the boundary between the top surface 12 and the front surface 13, and the boundary between the back surface 11 and the front surface 13 each have roundness at the corner (in other words, the rounded corner). Note that at least one of the boundary between the back surface 11 and the top surface 12, the boundary between the top surface 12 and the front surface 13, and the boundary between the back surface 11 and the front surface 13 need not have roundness at the corner.

The back surface 11 and the top surface 12 of the sole support 10, together with the top surface 21 of the sole part 20, constitute the support surface which supports the sole of the user.

In this example, as illustrated in Figs. 3 to 6, the top surface 12 of the sole support 10 is a plane that coincides with the horizontal plane.

As illustrated in Fig. 3, the top surface 12 constitutes a part of the support surface under at least a part (in this example, almost the whole) of the distal phalange BN1 at a higher position than a part (in this example, the top surface 21) of the support surface under the proximal joint of the proximal phalange BN3 so that the proximal joint of the distal phalange BN1 is above the proximal joint of the proximal phalange BN3 in the second toe FF2.

In each of the second toe FF2 to the fifth toe FF5, the proximal joint of the distal phalange BN1 may be represented as the distal interphalangeal (DIP) joint, the distal joint of the middle phalange BN2, or the joint between the distal phalange BN1 and the middle phalange BN2.

Also, in the first toe FF1, the proximal joint of the distal phalange BN1 may be represented as the interphalangeal (IP) joint, the distal joint of the proximal phalange BN3, or the joint between the distal phalange BN1 and the proximal phalange BN3.

In each of the first toe FF1 to the fifth toe FF5, the proximal joint of the proximal phalange BN3 may be represented as the metacarpophalangeal (MTP) joint, the distal joint of the metatarsal bone BN4, or the joint between the proximal phalange BN3 and the metatarsal bone BN4.

In each of the second toe FF2 to the fifth toe FF5, the proximal joint of the middle phalange BN2 may be represented as the proximal interphalangeal (PIP) joint, the distal joint of the proximal phalange BN3, or the joint between the middle phalange BN2 and the proximal phalange BN3.

In this example, similarly to the second toe FF2, the top surface 12 constitutes, for each of the first toe FF1 and the third toe FF3 to the fifth toe FF5, a part of the support surface under at least a part (in this example, almost the whole) of the distal phalange BN1 at a higher position than a part (in this example, the top surface 21) of the support surface under the proximal joint of the proximal phalange BN3 so that the proximal joint of the distal phalange BN1 is above the proximal joint of the proximal phalange BN3.

Thus, the top surface 12 of the sole support 10 constitutes, for each toe (in this example, each of the first toe FF1 to the fifth toe FF5), a part of the support surface under at least a part of the distal phalange BN1 at a higher position than a part of the support surface under the proximal joint of the proximal phalange BN3 so that the proximal joint of the distal phalange BN1 is above the proximal joint of the proximal phalange BN3 in each toe. Therefore, in this example, the top surface 12 of the sole support 10 corresponds to the high portion.

In this example, the top surface 12 of the sole support 10 is higher than a part (in this example, the top surface 21 of the sole part 20) of the support surface under the proximal joint of the proximal phalange BN3 by a certain top surface height for each toe. The top surface height may be preferably set according to the shape of the user's toes and the degree of the symptom of the user's toe deformity.

For example, the top surface height is 5 mm to 20 mm. By the way, when the top surface height is lower than 7 mm, the flexion of the proximal joint of the distal phalange BN1 may become excessive. Also, when the top surface height is higher than 15 mm, the distal end of the toe may slip off the top surface 12. Therefore, the top surface height is suitable to be 7 mm to 15 mm. In this example, the top surface height is approximately 10 mm.

In this example, the back edge of the top surface 12 has a position under the vicinity of the proximal joint of the distal phalange BN1 of each of the first toe FF1 to the fifth toe FF5.

As illustrated in Fig. 3, the back surface 11 constitutes a part, which is adjacent to the top surface 12 in the direction from the distal end of the second toe FF2 to the proximal end of the second toe FF2, of the support surface located on the proximal end side of the second toe FF2 with respect to the top surface 12 at a position becoming lower from the distal end of the second toe FF2 toward the proximal end of the second toe FF2. In this example, as illustrated in Fig. 3, the back surface 11 has a straight line tilted with respect to the horizontal plane in the cross section by the vertical plane extending along the second toe FF2.

In this example, similarly to the second toe FF2, for each of the first toe FF1 and the third toe FF3 to the fifth toe FF5, the back surface 11 constitutes a part, which is adjacent to the top surface 12 in the direction from the distal end of the toe to the proximal end of the toe, of the support surface located on the proximal end side of the toe with respect to the top surface 12 at a position becoming lower from the distal end of the toe toward the proximal end of the toe. In this example, similarly to the second toe FF2, for each of the first toe FF1 and the third toe FF3 to the fifth toe FF5, the back surface 11 has a straight line tilted with respect to the horizontal plane in the cross section by the vertical plane extending along the toe.

In this way, the back surface 11 of the sole support 10 constitutes, for each of the first toe FF1 to the fifth toe FF5 (in other words, each toe), a part, which is adjacent to the top surface 12 in the direction from the distal end of the toe to the proximal end of the toe, of the support surface located on the proximal end side of the toe with respect to the top surface 12 at a position becoming lower from the distal end of the toe toward the proximal end of the toe. Therefore, in this example, the back surface 11 of the sole support 10 corresponds to the change portion.

In this example, the top surface 12 and the back surface 11 of the sole support 10 form, for each toe in the cross section by the vertical plane extending along the toe, a certain tilt angle by a straight line (in other words, a straight line connecting the front edge of the top surface 12 and the back edge of the top surface 12) connecting the both ends of the top surface 12 and a straight line (in other words, a straight line connecting the upper edge of the back surface 11 and the lower edge of the back surface 11) connecting the both ends of the back surface 11. The tilt angle may be preferably set according to the shape of the user's toes and the degree of the symptom of the user's toe deformity.

For example, the tilt angle is an angle of 110 to 165 degrees. By the way, when the tilt angle is less than 125 degrees, the flexion of the proximal joint of the distal phalange BN1 may become excessive. Also, when the tilt angle is greater than 150 degrees, the proximal joint of the distal phalange BN1 may be extended excessively. Therefore, the tilt angle is suitable to be 125 to 150 degrees.

As described above, the sole support 10 of the first embodiment constitutes at least a part of a support surface that supports a sole. The sole support 10 includes a high portion (in this example, the top surface 12) that constitutes a part of the support surface under at least a part of a distal phalange BN1 at a higher position than a part of the support surface under a proximal joint of a proximal phalange BN3 for each toe so that a proximal joint of the distal phalange BN1 is above the proximal joint of the proximal phalange BN3 in each toe.

According to this, the proximal joint of the distal phalange BN1 of each toe has a position higher than the proximal joint of the proximal phalange BN3. This makes it possible to bring the distal joint of the proximal phalange BN3 for each toe closer to a state in which the distal joint is extended. Further, a ratio of a force applied to the tip portion of each toe to a total force applied to the sole can be reduced.

Thus, according to the sole support 10, an excessive force to be applied to the tip portion of each toe in a state where at least one of the distal joints of the proximal phalange BN3 of each toe is excessively bent can be avoided. Further, as compared with the case where the distal joint of the proximal phalange BN3 of each toe is fixed, the degree of freedom of motions of the distal joint and another joint in the vicinity of the distal joint can be increased. As a result, the user with the toe deformity can easily exercise by using the sole support 10.

Furthermore, in the sole support 10 of the first embodiment, under each of at least one toe of the second toe FF2 to the fifth toe FF5, an end (in this example, the back edge of the top surface 12) of the high portion in a direction from a distal end of the toe to a proximal end of the toe is closer to the distal end of the toe than the center of a middle phalange BN2 of the toe.

By the way, due to hammer toe, at least one of the distal joints of the middle phalanges BN2 of the second toe FF2 to the fifth toe FF5 may be extended excessively. On the other hand, according to the sole support 10, the distal joint of the middle phalange BN2 can be properly bent in the toe with hammer toe. Thus, in the toe with hammer toe, an excessive force to be applied to the tip portion of the toe in a state where the distal joint of the middle phalange BN2 is excessively extended can be avoided. As a result, the user with the toe deformity can easily exercise by using the sole support 10.

Furthermore, in the sole support 10 of the first embodiment, under each of at least one toe of the second toe FF2 to the fifth toe FF5, an end (in this example, the back edge of the top surface 12) of the high portion in a direction from a distal end of the toe to a proximal end of the toe is closer to the proximal end of the toe than a distal joint of a middle phalange BN2 of the toe.

By the way, due to claw toe, at least one of the distal joints of the middle phalanges BN2 of the second toe FF2 to the fifth toe FF5 may be bent excessively. On the other hand, according to the sole support 10, the distal joint of the middle phalange BN2 in the toe with claw toe can be brought closer to a state in which the distal joint is extended. Thus, in the toe with claw toe, an excessive force to be applied to the tip portion of the toe in a state where the distal joint of the middle phalange BN2 is excessively bent can be avoided. As a result, the user with the toe deformity can easily exercise by using the sole support 10.

Furthermore, the sole support 10 of the first embodiment includes a change portion (in this example, the back surface 11) that constitutes, for each toe, a part, which is adjacent to the high portion (in this example, the top surface 12) in a direction from a distal end of the toe to a proximal end of the toe, of the support surface located on a proximal end side of the toe with respect to the high portion at a position becoming lower from the distal end of the toe toward the proximal end of the toe.

By the way, the proximal joint of the proximal phalange BN3 of each toe may move closer to the distal end of the toe by sliding the part of the sole against the support surface with exercise. In this case, the distal joint of the proximal phalange BN3 of the toe may bend excessively.

On the other hand, according to the sole support 10, even if the part of the sole slides with respect to the support surface with exercise, the sole comes into contact with the change portion, so that the proximal joint of the proximal phalange BN3 of each toe can be suppressed to approach the distal end of the toe. Therefore, an excessive force to be applied to the tip portion of each toe in a state where at least one of the distal joints of the proximal phalange BN3 of each toe is excessively bent can be avoided. As a result, the user with the toe deformity can easily exercise by using the sole support 10.

In addition, in the sole support 10 of the first embodiment, the top surface 12 and the back surface 11 are continuous for each toe in a cross section by the vertical plane extending along the toe.

According to this, the area where the sole and the sole support 10 contact with each other can be increased. This allows a force applied to the sole to be distributed. Furthermore, the sole can be prevented from moving against the sole support 10. As a result, the user with the toe deformity can easily exercise by using the sole support 10.

Note that in the sole support 10 of a modified example of the first embodiment, the top surface 12 and the back surface 11 may be composed of multiple components, respectively. In this case, the top surface 12 and the back surface 11 may be separated from each other for each toe in the cross section by the vertical plane extending along the toe.

Also, in the sole support 10 of the first embodiment, for each toe, in the cross section by the vertical plane extending along the toe, a straight line (in other words, a straight line connecting the front edge of the top surface 12 and the back edge of the top surface 12) connecting the both ends of the top surface 12 extends in the horizontal direction.

This prevents the proximal joint of the distal phalange BN1 from extending excessively and the proximal joint of the distal phalange BN1 from bending excessively. As a result, the user with the toe deformity can easily exercise by using the sole support 10.

By the way, the sole support 10 of the first embodiment is solid. Note that the sole support 10 of a modified example of the first embodiment may be hollow. Alternatively, the sole support 10 of a modified example of the first embodiment may consist of a plate-shaped member, which constitutes the top surface 12 and the back surface 11, and a columnar member, which supports the plate-shaped member.

Alternatively, in the sole support 10 of a modified example of the first embodiment, each of the top surface 12 and the back surface 11 may be composed of multiple (e.g., many) components. In this case, a gap may be provided between the components. Further, in this case, each component may be a columnar member.

By the way, in the sole support 10 of the first embodiment, the back edge of the top surface 12 has a position more positive in the y-axis direction than the back edge of the bottom surface 14. In the sole support 10 of a modified example of the first embodiment, the back edge of the top surface 12 may have the same position in the y-axis direction as the back edge of the bottom surface 14, or have a position more negative in the y-axis direction than the back edge of the bottom surface 14.

By the way, in the sole support 10 of the first embodiment, the front edge of the top surface 12 has a position more positive in the y-axis direction than the front edge of the bottom surface 14. In the sole support 10 of a modified example of the first embodiment, the front edge of the top surface 12 may have the same position in the y-axis direction as the front edge of the bottom surface 14, or have a position more negative in the y-axis direction than the front edge of the bottom surface 14.

By the way, in the sole support 10 of the first embodiment, the top surface 12 coincides with the horizontal plane.

In the sole support 10 of a modified example of the first embodiment, the top surface 12 may be tilted with respect to the horizontal plane so that the front edge of the top surface 12 is higher than the back edge of the top surface 12. According to this, the proximal joint of the distal phalange BN1 can be brought further closer to a state in which the proximal joint is extended.

Alternatively, in the sole support 10 of a modified example of the first embodiment, the top surface 12 may be tilted with respect to the horizontal plane so that the front edge of the top surface 12 is lower than the back edge of the top surface 12. This prevents the distal end of the toe from slipping away from the top surface 12.

By the way, in the sole support 10 of the first embodiment, the top surface 12 is a plane.

In the sole support 10 of a modified example of the first embodiment, the top surface 12 may be curved for each toe so that the central portion is dent in the cross section by the vertical plane extending along the toe.

In the sole support 10 of the first embodiment, the back surface 11 has, for each toe, a straight line in the cross section by the vertical plane extending along the toe.

Note that in the sole support 10 of a modified example of the first embodiment, the back surface 11 may be curved for each toe so that the central portion is dent in the cross section by the vertical plane extending along the toe.

By the way, in the sole support 10 of the first embodiment, the back edge of the top surface 12 is under the vicinity of the proximal joint of the distal phalange BN1 in each of the first toe FF1 to the fifth toe FF5.

In the sole support 10 of a modified example of the first embodiment, the back edge of the top surface 12 may be positioned under each of at least one toe of the first toe FF1 to the fifth toe FF5 between the proximal joint of the distal phalange BN1 and the center of the distal phalange BN1. Alternatively, in the sole support 10 of a modified example of the first embodiment, the back edge of the top surface 12 may be positioned under the first toe FF1 between the center of the proximal phalange BN3 of the first toe FF1 and the proximal joint of the distal phalange BN1. Alternatively, in the sole support 10 of a modified example of the first embodiment, the back edge of the top surface 12 may be positioned under each of at least one toe of the second toe FF2 to the fifth toe FF5 between the center of the middle phalange BN2 of the toe and the proximal joint of the distal phalange BN1.

Alternatively, in the sole support 10 of a modified example of the first embodiment, the back edge of the top surface 12 may have a position (for example, a position near the proximal joint of the middle phalange BN2) under each of at least one toe of the second toe FF2 to the fifth toe FF5 closer to the proximal end of the toe than the distal joint of the middle phalange BN2 of the toe. In this case, for example, the back edge of the top surface 12 may have a position under each of the fourth toe FF4 and the fifth toe FF5 near the proximal joint of the middle phalange BN2.

The sole support 10 of a modified example of the first embodiment may have a partition wall between the toes on the top surface 12.

### <Second Embodiment>

Next, a sole support of a second embodiment will be described. The sole support of the second embodiment differs from the sole support of the first embodiment in that the top surface of the sole support has unevenness. The following is a description focusing on the differences. In the description of the second embodiment, the one with the same reference sign as used in the first embodiment is the same or substantially the same one.

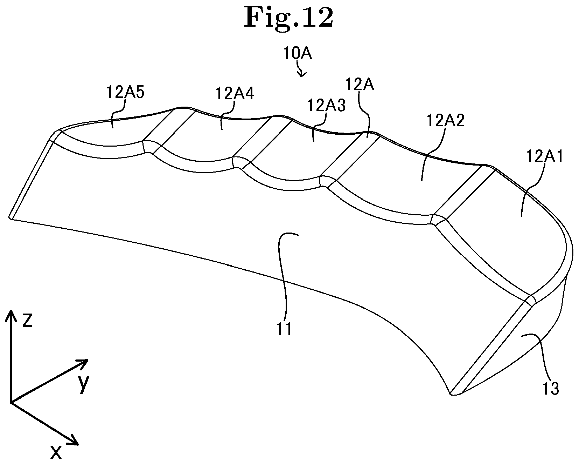

As illustrated in Figs. 12 to 18, the sole support 10A of the second embodiment includes a top surface 12A instead of the top surface 12 of the first embodiment.

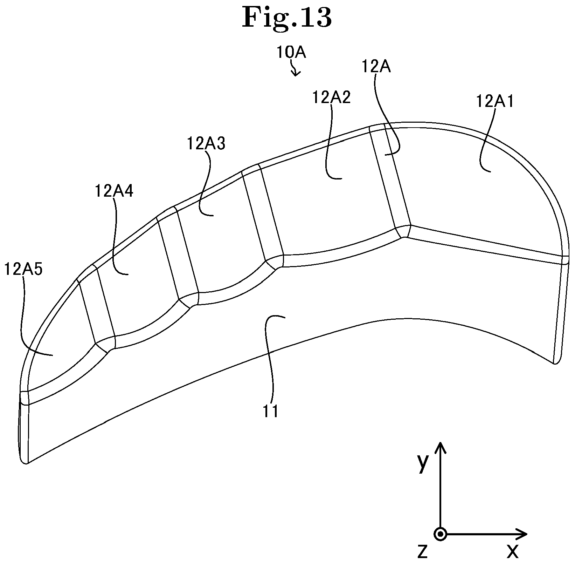

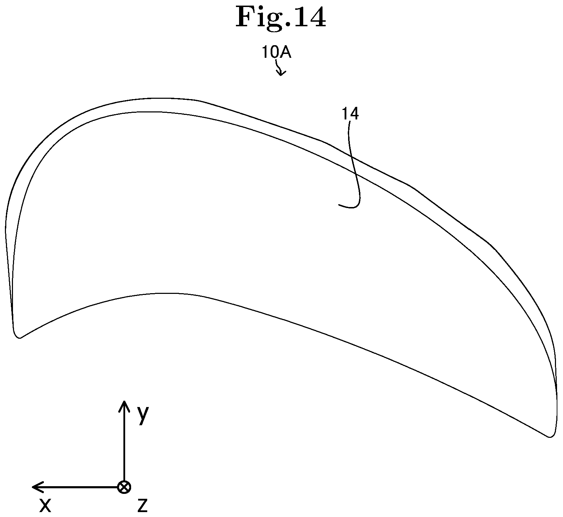

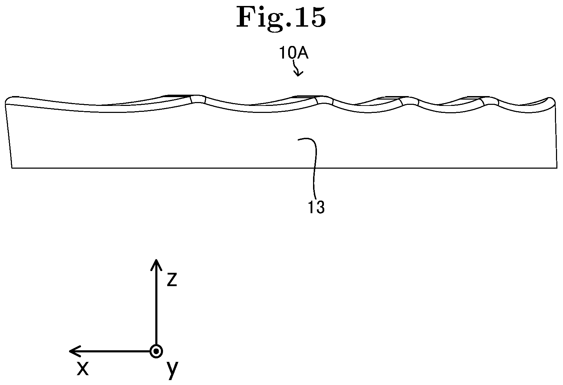

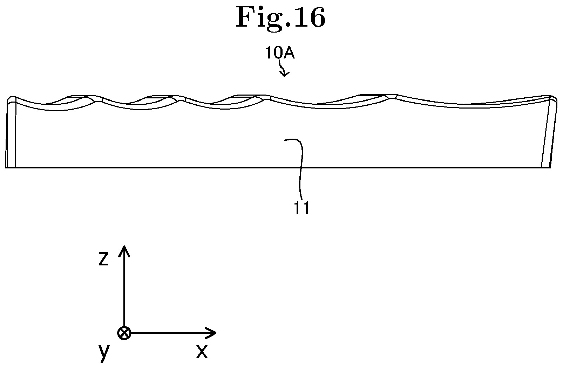

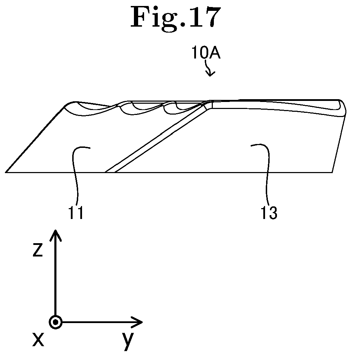

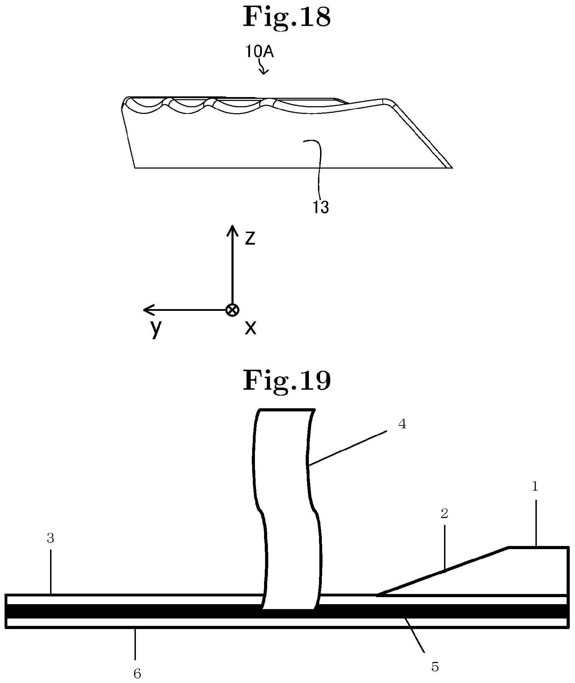

Fig. 12 is a right back upper perspective view of the sole support 10A. Fig. 13 is a plan view of the sole support 10A. Fig. 14 is a bottom view of the sole support 10A. Fig. 15 is a front view of the sole support 10A. Fig. 16 is a back view of the sole support 10A. Fig. 17 is a right side view of the sole support 10A. Fig. 18 is a left side view of the sole support 10A.

As illustrated in Figs. 12 and 13, the top surface 12A has a first recess 12A1, a second recess 12A2, a third recess 12A3, a fourth recess 12A4, and a fifth recess 12A5 located under the first toe FF1 to the fifth toe FF5, respectively.

The first recess 12A1 is curved so that, in the cross section by the vertical plane orthogonal to the direction along the first toe FF1, a part under the both end portions of the first toe FF1 is higher than a part under the central portion of the first toe FF1. Each of the second recess 12A2 to the fifth recess 12A5 is also configured similar to the first recess 12A1, except that they correspond to the second toe FF2 to the fifth toe FF5 instead of the first toe FF1, respectively.

As described above, according to the sole support 10A of the second embodiment, operations and effects similar to those of the sole support 10 of the first embodiment are accomplished.

Furthermore, in the sole support 10A of the second embodiment, in a cross section by a vertical plane orthogonal to a direction along a toe, the high portion (in this example, the top surface 12A) is curved so that a part of the high portion under both end portions of the toe is higher than a part of the high portion under a central portion of the toe.

According to this, the area where the sole and the sole support 10A contact with each other can be increased. This allows a force applied to the sole to be distributed. Furthermore, the toe can be prevented from moving against the sole support 10A. As a result, the user with the toe deformity can easily exercise by using the sole support 10A.

In the sole support 10A of a modified example of the second embodiment, the top surface 12A may have a recess only for each of the part of the first toe FF1 to the fifth toe FF5. In this case, for example, the top surface 12A may have a recess only for the fifth toe FF5, or may have a recess only for each of the fourth toe FF4 and the fifth toe FF5.

### <Third Embodiment>

Next, a sole support of a third embodiment will be described. In this example, the sole support may be referred to as a three-dimensional mallet toe corrector or a three-dimensional structure pad.

### (Technical Field)

The third embodiment is a corrector used for mallet toe correction, and more specifically, a three-dimensional mallet toe corrector that corrects a mallet toe by holding a joint of the toe in a state where the joint is extended.

### (Background Art)

The mallet toe (or, hammer toe) is a symptom with the toe being bent due to a various cause. This is accompanied by pain when the toe is pressed by standing or walking. For this reason, it is one of the biggest obstacles, especially when performing rehabilitation of paralyzed patients.

Proximal interphalangeal (PIP) joint and distal interphalangeal (DIP) joint of toes in human body are a kind of hinge joint, and motion is only extension and flexion. The improvement of the mallet toe symptom is expected by maintaining a state in which the flexion of the proximal interphalangeal (PIP) joint and the distal interphalangeal (DIP) joint, which are hinge joints, is restricted.

The conventional mallet toe corrector adopts a method that maintains the above state of extending the toe by pressing the joint from above with the sole in a plane-like state, or by fixing the toe with a belt or the like.

### (Technical Problem)

In the conventional mallet toe corrector, there are some cases where the flexion of the toe is not restricted and the mallet toe symptom is not improved depending on the strength of the bending force of the toe.

The third embodiment relates to a three-dimensional structure pad under the bottom of the toes for maintaining the state of extending the toes.

### (Solution to Problem)

That is, the third embodiment is a three-dimensional pad under a bottom of toes for a purpose of correcting the a toe shape having a tilted shape (2) under the bottom of the toes from metacarpophalangeal (MP) joints to an interphalangeal (IP) joint of a first toe and distal interphalangeal (DIP) joints, and a plane shape (1) from the interphalangeal (IP) joint of the first toe and the distal interphalangeal (DIP) joints to the tips of the toes.

### (Advantageous Effects)

Proximal interphalangeal joint (PIP) and distal interphalangeal joint (DIP) of toes in human body are forms of hinge joint, and motion is only extension and flexion.

In a state where the metacarpophalangeal joints are extended, the interphalangeal (IP) joint of the first toe and the distal interphalangeal (DIP) joints are extended, and the flexion of the tips of the toes is restricted, the proximal interphalangeal (PIP) joint and the distal interphalangeal (DIP) joint, which are hinge joints, are in a state where the flexion motion is extremely difficult due to its structure.

The above state of the toes is achieved by attaching the three-dimensional pad to the bottom of the toes. Thus, by restricting the flexion of the toes, the mallet toe symptom can be corrected.

Also, compared to the conventional method, the effect of the strength of the flexion of the toes can be reduced.

### (Configuration)

Next, the third embodiment will be described based on figures illustrating a specific example.

Fig. 19 is a side view of the three-dimensional mallet toe corrector.

The part of the sole from the interphalangeal (IP) joint of the first toe and the distal interphalangeal (DIP) joints to the tips of the toes is aligned with the upper part of a plane portion 1 in Fig. 19.

The bottom of the toes from the metacarpophalangeal (MP) joints to the interphalangeal (IP) joint of the first toe and the distal interphalangeal (DIP) joints contacts the top surface of a tilted portion 2 in Fig. 19. The bottom of the sole contacts the top surface of a plane portion 3 in Fig. 19.

In the above state, the foot and the three-dimensional mallet toe corrector are fixed with a foot fixing belt 4 in Fig. 19.

As described above, in a state where the bottom of the toes and the three-dimensional mallet toe corrector are fixed, the metacarpophalangeal joints are extended, the interphalangeal (IP) joint of the first toe and the distal interphalangeal (DIP) joints are extended, and the flexion of the tips of the toes is restricted, the proximal interphalangeal (PIP) joint and the distal interphalangeal (DIP) joint, which are hinge joints, are in a state where the flexion motion is extremely difficult due to its structure. Therefore, the effect of correcting the mallet toe symptom can be obtained.

In using the three-dimensional pad as the method, the present invention is not limited to the above-described embodiment. Design changes, that can be conceived by those skilled in the art, including various modifications and deformations without departing from the spirit of the present invention are included in the present invention.

### (Overview)

The mallet toe (or, hammer toe) is a symptom with the toe being bent due to a various cause. This is accompanied by pain when the toe is pressed by standing or walking. For this reason, it is one of the biggest obstacles, especially when performing rehabilitation of paralyzed patients.

In the conventional mallet toe corrector, there are some cases where the flexion of the toe is not restricted and the mallet toe symptom is not improved depending on the strength of the bending force of the toe.

By attaching a three-dimensional pad to the bottom of the toes, the toes are maintained to be extended. Thus, the flexion of the toes is restricted. This makes the correction of the mallet toe symptom possible.

Also, compared to the conventional method, the effect of the strength of the flexion of the toes can be reduced.

### <Note>

Further, the present invention may be expressed as follows.

### (Note 1)

A three-dimensional pad under a bottom of toes for a purpose of correcting a toe shape comprising:
a tilted shape (2) under the bottom of the toes from metacarpophalangeal (MP) joints to an interphalangeal (IP) joint of a first toe and distal interphalangeal (DIP) joints; and
a plane shape (1) from the interphalangeal (IP) joint of the first toe and the distal interphalangeal (DIP) joints to tips of the toes.

### (Note 2)

The three-dimensional pad for the purpose of correcting the toe shape according to note 1, wherein
the three-dimensional pad (1, 2, 3, 6) uses a rubber material with pressure resistance or a material with a similar effect, and has an effect of maintaining the toes to be extended.

### (Note 3)

The three-dimensional pad for the purpose of correcting the toe shape according to note 1, wherein
the three-dimensional pad has a shape continuous from a sole plate, uses a hard plastic material or a material (5) with a similar effect for a middle layer of a sole portion, and has an effect of maintaining strength of the sole portion.

### (Note 4)

The three-dimensional pad for the purpose of correcting the toe shape according to note 1, wherein
the three-dimensional pad has a foot fixing belt (4) attached to a hard plastic material of a middle layer of a sole portion or a material having a similar effect, and has an effect of fixing a foot and the three-dimensional pad.

The present invention is not limited to the above embodiment. For example, various modifications that can be understood by those skilled in the art may be added to the above embodiments without departing from the spirit of the present invention.

The present invention is based upon and claims the benefit of priority of the prior Japanese Patent application 2020-27567 filed on February 3, 2020, the entire contents of which are incorporated herein by reference.

### Reference Signs List

- 10, 10A: sole support
- 11: back surface
- 12, 12A: top surface
- 12A1: first recess
- 12A2: second recess
- 12A3: third recess
- 12A4: fourth recess
- 12A5: fifth recess
- 13: front surface
- 14: bottom surface
- 20: sole part
- 21: top surface
- FF: foot
- FF1: first toe
- FF2: second toe
- FF3: third toe
- FF4: fourth toe
- FF5: fifth toe
- BN1: distal phalange
- BN2: middle phalange
- BN3: proximal phalange
- BN4: metatarsal bone
- 1: three-dimensional pad plane portion
- 2: three-dimensional pad tilted portion
- 3: sole plane portion
- 4: foot fixing belt
- 5: sole portion hard material
- 6: bottom portion

## Claims

1. A sole support that constitutes at least a part of a support surface that supports a sole comprising
a high portion that constitutes a part of the support surface under at least a part of a distal phalange at a higher position than a part of the support surface under a proximal joint of a proximal phalange for each toe so that a proximal joint of the distal phalange is above the proximal joint of the proximal phalange in each toe.

2. The sole support according to claim 1, wherein
under each of at least one toe of second to fifth toes, an end of the high portion in a direction from a distal end of the toe to a proximal end of the toe is closer to the distal end of the toe than the center of a middle phalange of the toe.

3. The sole support according to claim 1 or 2, wherein
under each of at least one toe of second to fifth toes, an end of the high portion in a direction from a distal end of the toe to a proximal end of the toe is closer to the proximal end of the toe than a distal joint of a middle phalange of the toe.

4. The sole support according to any one of claims 1 to 3, further comprising
a change portion that constitutes, for each toe, a part, which is adjacent to the high portion in a direction from a distal end of the toe to a proximal end of the toe, of the support surface located on a proximal end side of the toe with respect to the high portion at a position becoming lower from the distal end of the toe toward the proximal end of the toe.

5. The sole support according to any one of claims 1 to 4, wherein
the high portion, in a cross section of the high portion cut by a vertical plane orthogonal to a direction along a toe, is curved so that a part of the high portion under both end portions of the toe is higher than a part of the high portion under a central portion of the toe.
